# EUROPEAN PATENT APPLICATION

(11) **EP 1 533 355 A1**
(43) Date of publication of application: **25.05.2005**
(21) Application number: 03738624.0
(22) Date of filing: 01.07.2003
(51) Int. Cl.: C09K 3/00, A61K 7/00, A61K 7/02, C07C 279/12

(54) **ULTRAVIOLET-ABSORBING COMPOSITION**

(30) Priority: 04.07.2002 JP 2002196242
(71) Applicant: Ajinomoto Co., Inc., Tokyo 104-8315 (JP)
(72) Inventor: HATTORI, Tatsuya, c/o AJINOMOTO CO., INC., Kawasaki-shi, Kanagawa 210-8681 (JP)
(74) Representative: Strehl Schübel-Hopf & Partner
(86) International application number: PCT/JP2003/008380
(87) International publication number: WO 2004/005422

(57) **Abstract**

Ultraviolet absorption materials comprising an amide-containing guanidine derivative of the following general formula (I): 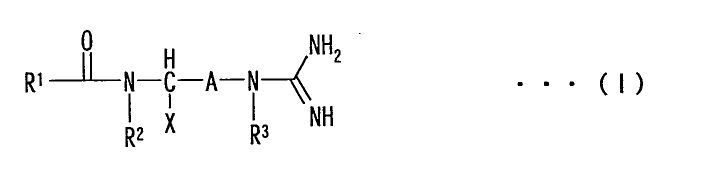 or salt thereof are disclosed. The materials have high solubility, and can be blended as ultraviolet absorption materials with a variety of cosmetics and adsorbed well on skin or hair.

## Description

### Technical Field

The present invention relates to novel water-soluble ultraviolet (UV) absorption compositions containing an amide-containing guanidine derivative or its salt.

### Background Art

So far, as a method for protecting skin or hair from ultraviolet ray, a UV scattering method using a metal oxide such as zinc oxide or titanium oxide or a method for absorbing UV with an organic compound such as a cinnamic acid-type or benzophenone-type compound has been employed.

The metal oxide, however, is not soluble in water or other solvents and further not adsorbed on skin or hair, and has been limited to some use such as foundation cosmetics, accordingly.

There was a problem that the organic UV absorbents, most of which are oil soluble, need addition of an emulsifying agent or the like when blended with aqueous cosmetics, giving an unfavorable feeling in use. There was another problem that the organic UV absorbent is washed away with sweat to decrease the expected effect of UV absorption, since it has no adsorption capability to skin or hair similarly in the metal oxide.

Therefore, it has been desired to develop a water-soluble UV absorbent which is readily adsorbed on skin or hair with resistance to rinsing-out.

On the other hand, the compounds having a guanidine group have been utilized in a wide variety of fields such as drugs, agrochemicals, bactericides, insecticides, valuable metal scavengers, chelating agents, and the like; in particular, the amide-containing guanidine derivatives or their salts are known to be used as surfactants as described in JP-A-2-243614/1990. It has not yet been known that a guanidine derivative or salt thereof to which an UV-absorbing group is attached through an amide linkage can be utilized as an UV absorbent.

### [Problems to be Solved by the Invention]

The purpose of the invention is to provide a water-soluble UV absorbing material which is readily adsorbed on hair or skin and is excellent in resistance to rinsing-out and an UV absorption composition utilizing the same.

### [Means for Solving the Problems]

In this situation, the present inventors worked assiduously to study a structural change of the so far known UV-absorbing materials and found that such a change produces a very soluble UV-absorbing material adsorbed well on skin and hair. Thus, the invention was completed.

### Disclosure of Invention

The invention comprises the following embodiments.
1) An ultraviolet absorption composition comprising at least one active ingredient selected from the group of amide-containing guanidine derivatives represented by the following general formula (I): 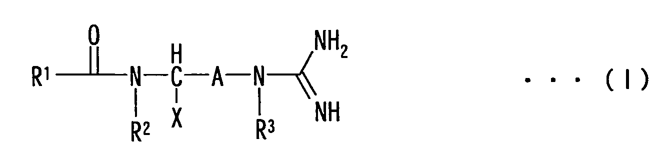 (wherein R¹ is a hydrocarbon group (it may contain a heteroatom on the carbon chain) showing a maximum absorption wavelength at 260 to 360nm in the form of R¹COOH and; R² and R³ each is independently a hydrogen atom or a straight or branched chain alkyl or alkenyl group of 1 to 4 carbon atoms; A is a straight or branched chain alkylene or alkenylene group of 1 to 10 carbon atoms; and x is a hydrogen atom or carboxyl group) and salts thereof.
2) An ultraviolet absorption composition comprisingat least one active ingredient selected from the group of amide-containing guanidine derivatives represented by the following general formula (II): 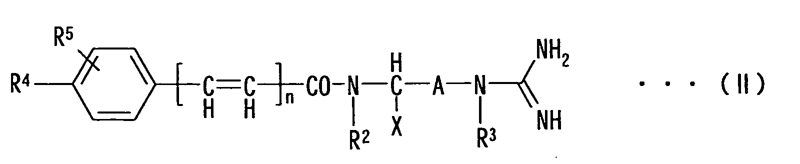 (wherein R² and R³ each is independently a hydrogen atom or a straight or branched chain alkyl or alkenyl group of 1 to 4 carbon atoms; R⁴ and R⁵ each is independently a hydrogen atom, halogen atom, hydroxyl group, alkyl group of 1 to 22 carbon atoms, alkoxy group of 1 to 22 carbon atoms, amino group, monoalkylamino group of 1 to 22 carbon atoms, or dialkylamino group of 1 to 22 carbon atoms; n is 0 or 1; A is a straight or branched chain alkylene or alkenylene group of 1 to 10 carbon atom; and x is a hydrogen atom or carboxyl group) and salts thereof.
3) An ultraviolet absorption composition as described in the above item 1), wherein R¹ in the above general general formula (I) is the same as R¹ in the form of R¹COOH selected from the group of cinnamic acid, p-hydroxycinnamic acid, p-alkoxycinnamic acid, 4-hydroxy-3-methoxycinnamic acid and 3,4-dialkoxycinnamic acid.
4) An ultraviolet absorption composition as described in any one of the above items 1) to 3), wherein in the above general formula (I) or (II) R², R³ and x are hydrogen atoms and A is a straight chain alkylene group of 3 carbon atoms.
5) An ultraviolet absorption composition as described in any one of the above items 1) to 3), wherein in the above general formula (I) or (II) R² and R³ are hydrogen atoms, x is a carboxyl group, and A is an alkylene group of 3 carbon atoms.
6) An ultraviolet absorption hair-care product, skin-care product or make-up cosmetic product characterized in that one or more member selected from the group of amide-containing guanidine derivatives of the above general formula (I) or (II) and salts thereof are blended.
7) An amide-containing guanidine derivative represented by the following general formula (III): 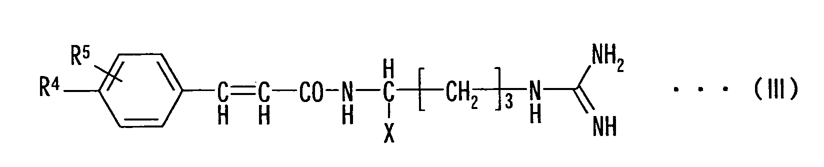 (wherein R⁴ and R⁵ each is independently a hydrogen atom, hydroxyl group, alkyl group of 1 to 22 carbon atoms, alkoxy group of 1 to 22 carbon atoms, amino group, monoalkylamino group of 1 to 22 carbon atoms, or dialkylamino group of 1 to 22 carbon atoms; and x is a hydrogen atom or carboxyl group; provided that the following cases are excluded: a case in which R⁴ and R⁵ are hydrogen atoms when x is a carboxyl group; a case in which R⁴ is a hydroxyl group and R⁵ is a hydrogen atom when x is a hydrogen atom; a case in which R⁴ is a methoxyl group and R⁵ is a hydrogen atom when x is a hydrogen atom; a case in which R⁴ is a hydrogen atom and R⁵ is a methoxyl group when x is a hydrogen atom; and a case in which R⁴ and R⁵ are methoxyl groups when x is a hydrogen atom) or salt thereof.

### Best Mode for Carrying Out the Invention

The invention will be explained in details as follows.

R¹ is a hydrocarbon group showing a maximum absorption wavelength at 260 to 360nm in the form of R¹COOH and may contain a heteroatom on the carbon chain. That is, R¹COOH per se is generally mentioned as a carboxylic acid-type UV-absorbing material having an aromatic ring. Specifically, it includes benzoic acids such as p-aminobenzoic acid, p-dialkylaminobenzoic acid, p-hydroxybenzoic acid, p-alkoxybenzoic acid, and the like, and cinnamic acid such as p-alkoxycinnamic acid, 4-hydroxy-3-alkoxycinnamic acid, and the like. In particular, as the starting material used in production of the UV absorbents (UV-absorbing materials) as active ingredients in the UV absorption compositions of the invention, a cinnamic acid represented by the above general formula (II) is preferably used in view of its easy availability and effectiveness; more preferably, cinnamic acid, p-hydroxycinnamic acid, p-alkoxycinnamic acid, 4-hydroxy-3-methoxycinnamic acid and 3,4-dialkoxycinnamic acid are included; and particularly preferred are cinnamic acid andp-alkoxycinnamic acid. In this connection, the UV absorbent represented by the above general formula (II) includes those compounds of the above general formula (I).

The wavelength showing the maximum absorption was measured by means of a UV spectrometer in a water and/or alcohol solution in a form of R¹COOH or its salt as mentioned in detail in Examples.

As for the acid used in formation of a salt of the amide-containing guanidine derivative, there is no particular limitation as far as it has no trouble in the contact with human bodies such as skin or hair. For example, inorganic acids (mineral acid) such as hydrochloric acid or sulfuric acid, organic acids such as citric acid, lactic acid or acetic acid, or amino acid compounds such as 2-pyrrolidone-5-carboxylic acid, glutamic acid or aspartic acid, are exemplified.

There is no particular limitation in production of the amide-containing guanidine derivatives or their salts of the invention, and they can be produced in a conventional way. According to the method as described in JP-A-6-312972/1994, for example, a guanidyl group can be introduced into a methyl ester of a carboxylic acid-type UV absorbing material through a monoamide-amine. The objective products can also be produced by a condensation reaction of an amino-containing guanidine derivative with an acid anhydride, alkyl ester or acid chloride of a carboxylic acid-type UV absorbent. The amino-containing guanidine derivative or its salt used in this reaction may be synthesized in a well-known method, for example, enzymatic reaction as described in Can. J. Chem., 1982, 60: 2810-2820, or chemical reaction as described in Z. PHYSIOL. CHEM 1910, 68: 170-172.

The ultraviolet absorption compositions of the invention containing an amide-containing guanidine derivative or its salt as active ingredient can be prepared by adding diluents such as water and/or alcohol to the amide-containing guanidine derivative or its salt (compositions in a narrow sense); this includes the amide-containing guanidine derivative or its salt per se of the invention (compositions in a broad sense).

The amide-containing guanidine derivatives or salts thereof have a ultraviolet absorption capability and can be blended with a variety of cosmetics. The cosmetic formulation is optional and includes solution systems, solubilization systems, emulsion systems, pulverization systems, and the like. Such cosmetic formulation may be used in hair-care products such as shampoo, hair rinse, hair conditioner, hair cream or hair setting preparations, skin-care products such as skin lotion, emulsion, cream or facial mask, or make-up cosmetic products such as foundation cosmetics, lip sticks or eye shadow. Particularly, when used in hair, the guanidine derivatives of the invention are highly effective to show a high persistency of UV absorption effect since they have a high adsorption capability to hair and resist to wash-out by rinsing, i.e., anti-rinsing properties. In other words, these amide-containing guanidine derivatives or salts thereof can be substituted for the known UV-absorbing materials (e.g., benzophenone derivatives, methoxycinnamic acid derivatives and salicylic acid derivatives).

The amide-containing guanidine derivatives or salts thereof of the invention can also be used as surface protective agents for textiles or paper from ultraviolet ray. In addition, they may be used industrially by adding as UV absorbents to water paint.

Though the amount to be blended depends on the kind of cosmetics to be blended, the guanidine derivative usually may be used in the range of 0.01 to 10 wt%, preferably 0.1 to 7 wt%, more preferably 0.5 to 7 wt%, relative to the solubility and its effect.

In addition to the above-mentioned diluents, it is possible to add a variety of additives used in this field to the ultraviolet absorption compositions containing the amide-containing guanidine derivatives or salts thereof used in the invention in such a degree that they have no adverse effect. Such additives include those conventionally used in cosmetics and dermal external preparations, for example, ionic surface active agents, non-ionic surface active agents, moist-retaining agents, powder, pigments, oils, anti-oxidants, thickeners, film forming agents, organic solvents, preservatives, chelating agents, perfumes, and the like.

In particular, the compounds represented by the above general formula (III) are novel compounds which are expected to have performance as UV absorbents as well as to be applicable to cosmetics and other utility, though they are included in the compounds represented by the above general formula (II).

In view of easy availability of the starting materials and the degree of UV absorption capability, it is appropriate to use the compounds of the above general formula (II) wherein R⁴ is a hydrogen atom or an alkoxy group of 1 to 18 carbon atoms, R⁵ is a hydrogen atom, and x is a hydrogen atom or carboxyl group (provided that the cases wherein R⁴ and R⁵ are hydrogen atoms, and x is a carboxyl group, and R⁴ is a methoxy group and R⁵ and x are hydrogen atoms, are excluded), more preferably, the compounds wherein R⁴ is a hydrogen atom or an alkoxy group of 1 to 12 carbon atoms, R⁵ is a hydrogen atom, and x is a hydrogen atom or a carboxyl group (provided that the case wherein R⁴ and R⁵ are hydrogen atoms, and x is a carboxyl group, and R⁴ is a methoxy group and R⁵ and x are hydrogen atoms, are excluded), and particularly the compounds wherein R⁴, R⁵ and x all are hydrogen atoms (the starting material, i.e., carboxylic acid-type UV-absorbing material, is cinnamic acid, which is bound to agmatine), wherein R⁴ is a methoxy group, R⁵ is a hydrogen atom, and x is a carboxyl group (the starting material, i.e., carboxylic acid-type UV-absorbing material, is p-methoxycinnamic acid, which is bound to arginine), and wherein R⁴ is a dodecyloxy group, R⁵ is a hydrogen atom, and X is a hydrogen atom or a carboxyl group (the raw material, i.e., carboxylic acid-type UV-absorbing material, isp-dodecyloxycinnamic acid, which is bound to arginine or agmatine).

### [Examples]

The following examples will illustrate specifically the present invention, but they are not intended to limit the invention.

### Preparation 1

### Synthesis of N-cinnamoylamidobutylguanidine 1/2 sulfate

### (Synthesis of Compound 1)

To a solution of 72.5 g (0.318 mol) of 4-aminobutylguanidine sulfate in 425 g of water is added 200 g of IPA, and the mixture is adjusted at pH 11 with 27% NaoH. There are added 51.3 g (0.308 mol) of cinnamoyl chloride and 27% NaOH while the mixture is kept at a temperature of 15°C and pH 11. After addition of cinnamoyl chloride, the mixture is stirred at the same temperature and pH for 30 minutes. The mixture is then warmed up to 40°C to separate into 2 layers. To the upper layer is added 300 g of water, and about 300 g of water/IPA is then distilled off under reduced pressure. The condensed liquid after addition of 250 g of water and cooling yields crystals as precipitate. The precipitate is washed with water to give 67.4 g of N-cinnamoylamidobutylguanidine 1/2 sulfate as crude crystals. These crude crystals are recrystallized from a mixture of IPA and water to give 60.7 g (0.196 mol; yield: 63.6%) of N-cinnamoylamidobutylguanidine 1/2 sulfate as white powder. The resulting compound is analyzed to give the following data.
¹H-NMR (CD₃OD) δ: 1.65 (br. 4H), 3.21 (m, 2H), 3.34 (m, 2H), 6.65 (d, 1H), 7.38 (m, 3H), 7.50 (d, 1H), 7.55 (m, 2H).
¹³C-NMR (DMSO-d6) δ: 157.39 (guanidine), 165.56 (amide)
ESI-Mass: 261 (MH+)

### Preparation 2

### Synthesis of N-cinnamoylamidobutylguanidine hydrochloride

### (Synthesis of Compound 2)

In a mixture of 13 g of IPA and 12 g of water was dissolved 3.0 g (9.7 mmol) of N-cinnamoylamidobutylguanidine 1/2 sulfate prepared in Preparation 1. There was added a solution of 1.2 g (4.9 mmol) of barium chloride dihydrate in 15 ml of water. The precipitate was filtered off, and the filtrate concentrated under reduced pressure to give 2.6 g (8.8 mmol) of N-cinnamoylamidobutylguanidine hydrochloride.

### Preparation 3

### Synthesis of N-paramethoxycinnamoylarginine

### (Synthesis of Compound 3)

To a suspension of 25.6 g (147.0 mmol) of arginine in 66 g of water and 153 g of t-BuOH is added a solution of 27.5 g (140.0 mmol) of paramethoxycinnamoyl chloride in toluene and 27% NaOH at 20°C and pH 10. After addition of paramethoxycinnamoyl chloride, the mixture is stirred at the same temperature and pH for 30 minutes. Then the reaction mixture is adjusted to pH 5 with addition of concentrated hydrochloric acid to give crystals as precipitate. The latter is washed with water and ethanol to give 15.3 g of crude crystals, which are recrystallized from a mixture of IPA and water to give 13.0g (38.9 mmol; yield: 27.8%) of pure N-paramethoxycinnamoylarginine as white powder. The resulting compound is analyzed to give the following data. ¹H-NMR (CD₃OD) δ: 1.66 (m, 2H), 1.77 (m, 1H), 1.93 (m, 1H), 3.22 (m, 2H), 3.81 (s, 3H), 4.42 (t, 1H), 6.57 (d, 1H), 6.92 (m, 2H), 7.44 (d, 1H), 7.49 (m, 2H).
¹³C-NMR (DMSO-d6) δ: 157.59 (guanidine), 164. 87 (amide), 176.67 (carbonyl)
ESI-Mass: 335 (MH⁺)

### Preparation 4

### Synthesis of N-paradodecyloxycinnamoylamidobutylguanidine 1/2 sulfate

### (Synthesis of Compound 4)

A solution of 11.5 g (50.4 mmol) of 4-aminobutylguanidine sulfate dissolved in 100 g of water and 95 g of t-BuOH is adjusted at pH 11 with 27% NaOH. A solution of 15.8 g (45.1 mmol) of paradodecyloxycinnamoyl chloride dissolved in toluene and 27% NaOH are added thereto to maintain a pH of 11 at 25°C. After addition of paradodecyloxycinnamoyl chloride, the mixture is stirred at the same temperature and pH for 30 minutes. The reaction mixture is then adjusted at pH 3 with addition of 75% sulfuric acid to give crystals as precipitate. The latter is washed with water, ethanol and chloroform, and then recrystallized from a mixture of water and t-BuOH to give 6.7 g (13.6 mmol; yield: 30.1%) of pure N-paradodecyloxycinnamoylamidobutylguanidine 1/2 sulfate as white powder. The resulting compound is analyzed to give the following data.
¹H-NMR (CD₃OD) δ: 0.89 (t, 3H), 1.29 (br, 16H), 1.47 (m, 2H), 1.64 (br, 4H), 1.77 (m, 2H), 3.20 (m, 2H), 3.33 (m, 2H), 3.99 (t, 2H), 6.51 (d, 1H), 6.91 (m, 2H), 7.46 (d, 1H), 7.50 (m, 2H).
ESI-Mass: 445 (MH⁺)

### Preparation 5

### Synthesis of N-paradodecyloxycinnamoylamidobutylguanidine hydrochloride

### (Synthesis of Compound 5)

In the same manner as in Preparation 2, the reaction was conducted using 3.0 g (6.1 mmol) of N-paradodecyloxycinnamoylamidobutylguanidine 1/2 sulfate of Preparation 4 in place of 3.0 g (9.7 mmol) of N-cinnamoylamidobutylguanidine 1/2 sulfate and using a mixture of 36 g of t-BuOH and 38 g of water in place of a mixture of 13 g of IPA and 12 g of water to give 2.5 g (5.2 mmol) of N-paradodecyloxycinnamoylamidobutylguanidine hydrochloride. Preparation 6

### Synthesis of N-paradodecyloxycinnamoylarginine

### (Synthesis of Compound 6)

To a suspension of 8.3 g (47.6 mmol) of arginine in 80 g of water and 103 g of t-BuOH is added a solution of 15.0 g (42.8 mmol) of paradodecyloxycinnamoyl chloride in toluene and 27% NaOH at 25°C and pH 10. After addition of paradodecyloxycinnamoyl chloride, the mixture is stirred at the same temperature and pH for 30 minutes. Then the reaction mixture is adjusted to pH 3 with addition of concentrated sulfuric acid to give crystals as precipitate. The latter is washed with water and t-BuOH, and then recrystallized from a mixture of water and t-BuOH to give 7.3 g (15.0 mmol; yield: 35.0%) of pure N-paradodecyloxycinnamoylarginine as white powder. The resulting compound is analyzed to give the following data.
¹H-NMR (CD₃OD) δ: 0.89 (t, 3H), 1.29 (br, 16H), 1.47 (m, 2H), 1.65 (m, 2H), 1.78 (m, 3H), 1.93 (m, 1H), 3.22 (m, 2H), 4.00 (t, 2H), 4.42 (t, 1H), 6.58 (d, 1H), 6.91 (m, 2H), 7.46 (d, 1H), 7.49 (m, 2H).
ESI-Mass: 489 (MH⁺)

### Test 1

### (Wavelength at the maximum absorption)

Table 1 shows the wavelength at the UV absorption wavelength and the molar absorption coefficients measured for the amide-containing guanidine derivatives obtained in Preparations and the starting carboxylic acid UV absorbents in water/alcohol solutions (30% methanol solution for Preparations 1 and 3; 40% isopropyl alcohol solution for Preparations 4 and 6).

**Table 1**

| | Amide-containing guanidine derivative | | Starting UV absorbent | |
|---|---|---|---|---|
| | Wavelength (nm) | Mol.Abs.Coef. (ε) | Wavelength (nm) | Mol.Abs.Coef. (ε) |
| Prep.1 | 280 | 21885 | 279 | 18661 |
| Prep.3 | 307 | 24977 | 307 | 20845 |
| Prep.4 | 307 | 24122 | 309 | 21246 |
| Prep.6 | 308 | 25740 | as above | as above |

As shown in Table 1, it is found that the absorption wavelength and the molar absorption coefficient of the amide-containing guanidine derivative are approximately the same as those of the starting UV absorbent carboxylic acid. Test 2

### (Solubility)

Table 2 shows the solubility of the amide-containing guanidine derivatives and of the starting carboxylic acid-type UV absorbents in water at room temperature.

**Table 2**

| | Amide-containing guanidine derivative (wt%) | Starting UV absorbent (wt%) |
|---|---|---|
| Compound 2 | >15 | <0.5 |
| Compound 3 (HCl salt) | >15 | <0.1 |
| Compound 5 | >7 | <0.01 |

As shown in Table 2, it is found that the conversion into the guanidine derivatives increase the solubility in water. Test 3

### (Adsorption on hair)

The adsorptive properties of Compound 1 as mentioned above as the amide-containing guanidine derivative or its salt of the invention and of cinnamidopropyl trimethylammonium chloride (INCROQUAT UV-283/CRODA) as a reference material were investigated on hair.

Experimental method: To a solution of 5 mg of Compound 1 in 30 ml of water is added 0.3 g of hair (5 mm in length) pretreated by bleaching, and the mixture is stirred at 40°C for 30 minutes. Before and after addition of the hair, Compound 1 in the aqueous solution is determined by HPLC, and the loss in the solution is regarded as the amount adsorbed on the hair.

Result: Compound 1 was adsorbed on hair at a proportion of about 3.5 mmol per 100 g of hair. The reference material was adsorbed at a proportion of about 1.7 mmol to the contrary. Blending 1

Using Compound 2 prepared in Preparation 2, a hair rinse product (hair care cosmetic) was prepared according to the prescription as shown in the following table 3.

**Table 3**

| | | |
|---|---|---|
| Component A | | |
| | Cetostearyl alcohol | 3.0 wt% |
| | Octyldodecyl myristate | 1.0 |
| | Glyceryl monostearate | 1.0 |
| Component B | | |
| | Monostearyltrimethylammonium chloride (50%) | 2.0 |
| | Compound 2 | 2.0 |
| | 1,3-Butylene glycol | 3.0 |
| | Antiseptic agent | adequate amt. |
| | Purified water | residual |
| | Total | 100.0 |

That is, Component A and Component B are respectively dissolved at 80°C, and Component A is added to Component B to form an emulsion. This was then cooled to 30°C to give a product.

The resulting hair rinse product gives an excellent feeling, and after drying it gives a wet feel and care of hair becomes easy.

### Blending 2

Using Compound 5 prepared in Preparation 5, a hair rinse product (hair care cosmetic) was prepared according to the prescription as shown in the following table 4.

**Table 4**

| | | |
|---|---|---|
| Component A | | |
| | Cetostearyl alcohol | 3.0 wt% |
| | Octyldodecyl myristate | 1.0 |
| | Glyceryl monostearate | 1.0 |
| Component B | | |
| | Monostearyltrimethylammonium chloride (50%) | 2.0 |
| | Compound 5 | 1.0 |
| | 1,3-Butylene glycol | 3.0 |
| | Antiseptic agent | adequate amt. |
| | Purified water | residual |
| | Total | 100.0 |

That is, Component A and Component B are respectively dissolved at 80°C, and Component A is added to Component B to form an emulsion. This was then cooled to 30°C to give a product.

The resulting hair rinse product gives an excellent feeling, and after drying it gives a light finishing feel and care of hair becomes easy.

### Blending 3

Using Compound 2 prepared in Preparation 2, a skin lotion (skin care cosmetic) was prepared according to the prescription as shown in the following table 5.

**Table 5**

| | |
|---|---|
| Sodium DL-pyrrolidonecarboxylate (50%) | 3.0 wt% |
| Sorbitol (70%) | 3.0 |
| Compound 2 | 7.0 |
| Carboxymethylcellulose | 0.3 |
| Antiseptic agent | adequate amt. |
| Purified water | residual |
| Total | 100.0 |

That is, each component was mixed and dissolved at 70-80°C with stirring. The mixture was cooled to 30°C with stirring to give a product.

The resulting skin lotion gave a good feeling without stickiness.

### Blending 4

Using Compound 3 prepared in Preparation 3, a hair gel product (hair care cosmetic) was prepared according to the prescription as shown in the following table 6.

| | |
|---|---|
| Table 6 | |
| Carboxyvinylpolymer | 0.7 wt% |
| Polyvinylpyrrolidone | 2.0 |
| Glycerin | 2.0 |
| Ethanol | 25.0 |
| Sodium hydroxide | adequate amt. |
| Compound 3 | 3.0 |
| Polyoxyethylene octyl dodecyl ether | 0.3 |
| Antiseptic agent | adequate amt. |
| Purified water | residual |
| Total | 100.0 |

That is, carboxyvinylpolymer was dispersed in glycerin and a part of purified water, to which was added the other components with stirring to give a product.

The resulting hair gel product was rich in setting potency and gave a good feeling without stickiness.

### Blending 5

Using Compound 6 prepared in Preparation 6, a hair shampoo product (hair care cosmetic) was prepared according to the prescription as shown in the following table 7.

**Table 7**

| | |
|---|---|
| Coconut oil fatty acid acylglutamic acid triethanolamine solution (30%) | 10.0 wt% |
| Polyoxyethylene lauryl ether sodium sulfate solution (27%) | 26.0 |
| Coconut oil fatty acid diethanolamide | 5.0 |
| Propylene glycol | 5.0 |
| Compound 6 | 1.5 |
| Cationic cellulose | 0.3 |
| Antiseptic agent | adequate amt. |
| Purified water | residual |
| Total | 100.0 |

That is, each component was mixed and dissolved at 70-80°C with stirring. The mixture was cooled to 30°C with stirring to give a product.

Using the resulting hair shampoo product , care of hair after drying becomes easy.

### Industrial Applicability

The amide-containing guanidine derivatives or salts thereof of the invention have a UV absorption power, are soluble in water and readily adsorbed onto skin or hair, and resist to rinsing. They accordingly can be blended with hair-care cosmetic products, particularly, of wash-out type, such as shampoo or hair rinse. Further, they can be blended with skin care cosmetic products, particularly, skin lotion, since they are highly soluble in water.

## Claims

1. An ultraviolet absorption composition comprising at least one active ingredient selected from the group of amide-containing guanidine derivatives represented by the following general formula (I): 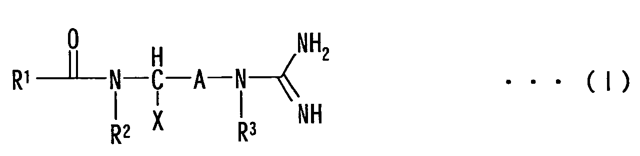 (wherein R¹ is a hydrocarbon group (it may contain a heteroatom on the carbon chain) showing a maximum absorption wavelength at 260 to 360nm in the form of R¹COOH and; R² and R³ each is independently a hydrogen atom or a straight or branched chain alkyl or alkenyl group of 1 to 4 carbon atoms; A is a straight or branched chain alkylene or alkenylene group of 1 to 10 carbon atoms; and X is a hydrogen atom or carboxyl group) and salts thereof.

2. Anultraviolet absorption composition comprising at least one active ingredient selected from the group of amide-containing guanidine derivatives represented by the following general formula (II): 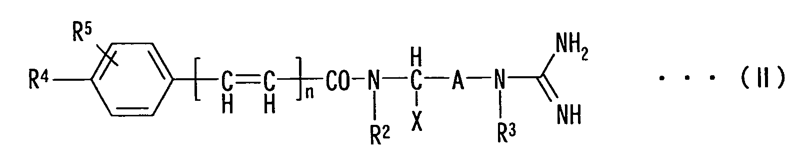 (wherein R² and R³ each is independently a hydrogen atom or a straight or branched chain alkyl or alkenyl group of 1 to 4 carbon atoms; R⁴ and R⁵ each is independently a hydrogen atom, halogen atom, hydroxyl group, alkyl group of 1 to 22 carbon atoms, alkoxy group of 1 to 22 carbon atoms, amino group, monoalkylamino group of 1 to 22 carbon atoms, or dialkylamino group of 1 to 22 carbon atoms; n is 0 or 1; A is a straight or branched chain alkylene or alkenylene group of 1 to 10 carbon atom; and x is a hydrogen atom or carboxyl group) and salts thereof.

3. An ultraviolet absorption composition as claimed in Claim 1, wherein R¹ in the above formula (I) is the same as R¹ in the form of R¹COOH selected from the group of cinnamic acid, p-hydroxycinnamic acid, p-alkoxycinnamic acid, 4-hydroxy-3-methoxycinnamic acid and 3,4-dialkoxycinnamic acid.

4. An ultraviolet absorption composition as claimed in any one of Claims 1 to 3, wherein in the above general formula (I) or (II) R², R³ and x are hydrogen atoms and A is a straight chain alkylene group of 3 carbon atoms.

5. An ultraviolet absorption composition as claimed in any one of Claims 1 to 3, wherein in the above general formula (I) or (II) R² and R³ are hydrogen atoms, x is a carboxyl group, and A is an alkylene group of 3 carbon atoms.

6. An ultraviolet absorption hair-care product, skin-care product or make-up cosmetic product **characterized in that** one or more member selected from the group of amide-containing guanidine derivatives of the above general formula (I) or (II) and salts thereof are blended.

7. An amide-containing guanidine derivative represented by the general formula (III): 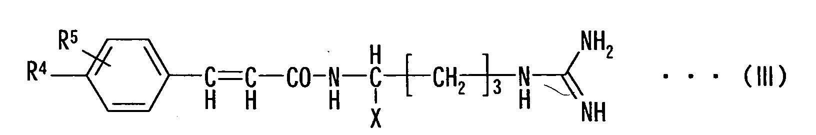 (wherein R⁴ and R⁵ each is independently a hydrogen atom, hydroxyl group, alkyl group of 1 to 22 carbon atoms, alkoxy group of 1 to 22 carbon atoms, amino group, monoalkylamino group of 1 to 22 carbon atoms, or dialkylamino group of 1 to 22 carbon atoms; and x is a hydrogen atom or carboxyl group; provided that the following cases are excluded: a case in which R⁴ and R⁵ are hydrogen atoms when x is a carboxyl group; a case in which R⁴ is a hydroxyl group and R⁵ is a hydrogen atom when x is a hydrogen atom; a case in which R⁴ is a methoxyl group and R⁵ is a hydrogen atom when x is a hydrogen atom; a case in which R⁴ is a hydroxyl group and R⁵ is a methoxyl group when x is a hydrogen atom; and a case in which R⁴ and R⁵ are methoxyl groups when x is a hydrogen atom) or salt thereof.
